# EUROPEAN PATENT APPLICATION

(11) **EP 4 338 785 A2**
(43) Date of publication of application: **20.03.2024**
(21) Application number: 23193420.9
(22) Date of filing: 25.08.2023
(51) Int. Cl.: A61M 39/22

(54) **DEVICE, SYSTEM AND METHOD FOR IN-SITU CALIBRATION OF BIOSENSORS**

(30) Priority: 26.08.2022 US 202263373623 P
(71) Applicant: Nerv Technology Inc., Kitchener, ON N2H 5L6 (CA)
(72) Inventor: Tjandra, Ricky, Kitchener, N2E 2A4 (CA); Berry, Khaled, Oakville, L6H 6H7 (CA); El-Falou, Abdallah Hassen, Kitchener, N2M 4N7 (CA); LeSergent, Lauren Janine, Waterloo, ON N2L4C3 (CA)
(74) Representative: De Clercq & Partners

(57) **Abstract**

Disclosed herein are devices, methods, and systems relating to in-situ calibration of biosensors. In an embodiment, a multiport stopcock device comprises: one or more inlet ports, receiving fluid from a fluid source, fluidically connected by a flow cell comprising one or more fluid channels, to one or more outlet ports; and a mechanism for diverting flow of fluid between ports and fluid channels, wherein: at least one of the one or more inlet ports comprises an inlet tip, connectable to a syringe, and a slip tip, connectable to said fluid source, and at least one of the one or more outlet ports comprises an inlet tip, connectable to a syringe, and a slip tip, connectable to a reservoir. Computer implemented methods of in-situ calibration of biosensors are disclosed. The devices, systems, and methods disclosed herein for in-situ calibration, may preferably decrease the risk of infection in patients requiring biofluid monitoring.

## Description

### CROSS-REFERENCE TO RELATED APPLICATIONS

This application claims priority from U.S. Provisional Patent Application No. 63/373, 623, filed August 26, 2022, the disclosure of which is hereby incorporated herein in its entirety by reference.

### FIELD OF THE INVENTION

The present invention relates generally to the field of calibration of biosensors, specifically to a device and system that enables in-situ calibration, more specifically to a method for the in-situ calibration of biosensors.

### BACKGROUND

Sensors are often used to monitor important biomarkers in patients' physiological fluids to assess their health status. It is advantageous to have a sensor that can measure the biomarkers in-situ, without having to draw samples or disconnect the sensor from the patient, due to a variety of reasons such as infection control. It is due to this need that several technologies have emerged to enable in-situ, bedside monitoring of a vast number of patient biomarkers.

One of the challenges for in-situ biomarker monitoring sensors is the need for biosensors to be calibrated periodically in order to maintain their accuracy. The typical calibration process involves removal of the sensor from the patient, exposure to a known reference material and re-attachment to the patient again.

A similar process would have to be followed if a validation procedure was to be done to determine whether or not the sensor is still functioning correctly. This need for removal during calibration/validation increases the risk of infection and decreases the value proposition of having in-situ monitoring in the first place.

In the prior art, there are disclosed various forms of medical grade stopcocks, some with fluid controlling devices, but none are coupled with biosensors which may be calibrated in-situ.

In the prior art, there are disclosed automatic stopcock actuators, for automatically rotating a stopcock handle in order to divert flow. However, the stopcocks do not enable fluid flow from a patient through the stopcock, nor a combination of selectable fluids, such as calibration, cleaning, or patient biofluids.

For example, US 10561832 discloses a medical stopcock comprising a body provided with three female connectors and a male connector, at least one of the female connectors being designed to receive an injection syringe, and a mobile plug which is mounted in the body. The stopcock can allow or block fluid flow from a syringe to a patient. However the device does not enable fluid flow from a patient through the stopcock, nor a combination of selectable fluids, such as calibration, cleaning, or patient biofluids.

US7695445 discloses a three-way stopcock that can be opened/closed in the same manner as conventional operation methods and can reduce stagnated portions of fluid in a fluid flow passage. Out of branch openings, a first branch opening and a second branch opening are arranged on a straight line, and a third branch opening is directed perpendicularly to the line connecting the first and second branch openings. Provided at a flow passage switch portion are a first flow-passage opening and a second flow-passage opening that are arranged on a straight line and a third flow-passage opening that is directed perpendicularly to the line connecting the first and second bran openings. The stopcock is not equipped with multiple channels, nor is it equipped with biosensors which may be calibrated in-situ. The stopcock may also not be automatically opened/closed.

In terms of in-situ calibration of devices, there exist some examples in the prior art of devices that "self calibrate"- however, they generally are not equipped to receive continuous flows of fluid from a patient, and are not capable of being calibrated for more than one analyte (i.e. only glucose, or only pH).

DE19715441 discloses in-situ calibration of chemosensors or biosensors immersed in sample solution, with one or more standard solutions. The sensor layer, with a reciprocal effect, is within the inner tube of a twin-tube system in direct contact with the sample for analysis. This is passed in pulses or continuously into the outer tube to be mixed with the standard solution in a fixed time sequence which can be repeated to show a constant or graduated concentration. The signals from the sensor show the actual sample fluid concentration, and the calibration function is computed. The calibration method does not disclose methods of calibrating sensors automatically, nor does it disclose calibration of sensors with a multichannel/multi-port flow cell/stopcock device, which enables on-site connection to the patient while calibrating medical sensors.

In an example, US9089292 and *https:*//*www.medtronicdiabetes.com*/*customer-supportlsensors-and-transmitters-supportlcalibration-sensor:* Continuous glucose monitoring may continuously monitor glucose while calibrating a monitor (i.e. for users with diabetes). However, they generally rely on external reference materials (such as a finger prick, to calibrate the glucose monitor), and it is not configured to receive continuous flow of fluids from a patient.

In another example, *http:*//*www.anbsensors.com*/*calibration-free-ph-sensor*/*:* Some prior art samples comprise built-in reference materials allowing for in-situ calibration. This generally only applies to pH sensors, and is not compatible with continuously flowing patient biofluids.

All documents cited herein are incorporated by reference.

None of the above cited documents, alone or in combination satisfy the need for a device, method, and system that enables automatic, and/or remote, in-situ calibration of biosensors.

This background information is provided to reveal information believed by the applicant to be of possible relevance. No admission is necessarily intended, nor should be construed, that any of the preceding information constitutes prior art or forms part of the general common knowledge in the relevant art.

### BRIEF SUMMARY

The following presents a simplified summary of the general inventive concept(s) described herein to provide a basic understanding of some aspects of the disclosure. This summary is not an extensive overview of the disclosure. It is not intended to restrict key or critical elements of embodiments of the disclosure or to delineate their scope beyond that which is explicitly or implicitly described by the following description and claims.

It is an object of the invention to provide a system and method for in-situ calibration of biosensors.

In accordance with an aspect, there is provided a multiport stopcock device comprising: one or more inlet ports, receiving fluid from a fluid source, fluidically connected by a flow cell comprising one or more fluid channels, to one or more outlet ports; and a mechanism for diverting flow of fluid between ports and fluid channels, wherein: at least one of the one or more inlet ports comprises an inlet tip, connectable to a syringe, and a slip tip, connectable to said fluid source, and at least one of the one or more outlet ports comprises an inlet tip, connectable to a syringe, and a slip tip, connectable to a reservoir.

In accordance with a second aspect, there is provided a method for in-situ calibration of a multi-port stopcock flow cell, the method comprising: executing, by a computing device, instructions stored on the memory, which cause the processor to perform the steps:
1. Prompt a user to insert a calibration fluid into an input port of a multiport stopcock device, the calibration fluid flow diverted via a mechanism for diverting flow, through a flow cell comprising one or more fluid channels, the flow cell fluidically connected to the multiport stopcock device, the multiport stopcock device comprising one or more sensors for measuring data relating to the calibration fluid;
2. Receive, via a connection mechanism connecting the one or more sensors to the computing device, the measured data;
3. Determine whether the measured data is valid, and upon the data being valid, repeat steps 1-3 until the user indicates that there are no more calibration fluids;
4. Filter the calibration data based on pre-determined thresholds.

In accordance with a third aspect, there is provided a computer readable, non-transitory storage medium, comprising instructions that, when executed, perform the steps:
1. Prompt a user to insert a calibration fluid into an input port of a multiport stopcock device, the calibration fluid flow diverted via a mechanism for diverting flow, through a flow cell fluidically connected to the multiport stopcock device, comprising one or more sensors for measuring data relating to the calibration fluid;
2. Receive, via a connection mechanism connecting the one or more sensors to the computing device, the measured data;
3. Determine whether the measured data is valid, and upon the data being valid, repeat steps 1-3 until the user indicates that there are no more calibration fluids;
4. Filter the calibration data based on pre-determined thresholds.

The advantages and features of the present invention will become better understood with reference to the following more detailed description and claims taken in conjunction with the accompanying drawings in which like elements are identified with like symbols.

To easily identify the discussion of any particular element or act, the most significant digit or digits in a reference number refer to the figure number in which that element is first introduced.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 illustrates a top view of the stopcock in accordance with one embodiment.
FIG. 2 illustrates a side view of the stopcock matter in accordance with one embodiment.
FIG. 3 illustrates a cross-sectional view of the stopcock in accordance with one embodiment.
FIG. 4 illustrates a rear view of the stopcock in accordance with one embodiment.
FIG. 5 illustrates an aspect of a four-way stopcock in accordance with one embodiment.
FIG. 6 illustrates an aspect of a four-way stopcock in accordance with one embodiment.
FIG. 7 illustrates an aspect of a four-way stopcock in accordance with one embodiment.
FIG. 8A illustrates a method of in-situ calibration of biosensors, in accordance with one embodiment.
FIG. 8B illustrates a method of in-situ calibration of biosensors, in accordance with one embodiment.
FIG. 8C illustrates a method of in-situ calibration of biosensors, in accordance with one embodiment.

Elements in the several drawings are illustrated for simplicity and clarity and have not necessarily been drawn to scale. For example, the dimensions of some of the elements in the figures may be emphasized relative to other elements for facilitating understanding of the various presently disclosed embodiments. Also, common, but well-understood elements that are useful or necessary in commercially feasible embodiments are often not depicted in order to facilitate a less obstructed view of these various embodiments of the present disclosure.

### DETAILED DESCRIPTION

Various implementations and aspects of the specification will be described with reference to details discussed below. The following description and drawings are illustrative of the specification and are not to be construed as limiting the specification. Numerous specific details are described to provide a thorough understanding of various implementations of the present specification. However, in certain instances, well-known or conventional details are not described in order to provide a concise discussion of implementations of the present specification.

Furthermore, numerous specific details are set forth in order to provide a thorough understanding of the implementations described herein. However, it will be understood by those skilled in the relevant arts that the implementations described herein may be practiced without these specific details. In other instances, well-known methods, procedures and components have not been described in detail so as not to obscure the implementations described herein.

In this specification, elements may be described as "configured to" perform one or more functions or "configured for" such functions. In general, an element that is configured to perform or configured for performing a function is enabled to perform the function, or is suitable for performing the function, or is adapted to perform the function, or is operable to perform the function, or is otherwise capable of performing the function.

When introducing elements of aspects of the disclosure or the examples thereof, the articles "a," "an," "the," and "said" are intended to mean that there are one or more of the elements. The terms "comprising," "including," and "having" are intended to be inclusive and mean that there may be additional elements other than the listed elements. The term "exemplary" is intended to mean "an example of." The phrase "one or more of the following: A, B, and C" means "at least one of A and/or at least one of B and/or at least one of C."

Devices and methods for carrying out the invention are presented in terms of embodiments depicted within the FIGS. However, the invention is not limited to the described embodiments, and a person skilled in the art will appreciate that many other embodiments of the invention are possible without deviating from the basic concept of the invention, and that any such work around will also fall under scope of this invention. It is envisioned that other styles and configurations of the present invention can be easily incorporated into the teachings of the present invention, and the configurations shall be shown and described for purposes of clarity and disclosure and not by way of limitation of scope.

According different embodiments, there is disclosed a multi-port stopcock device, system, and/or process for use in in-situ monitoring and/or calibration of biofluids.

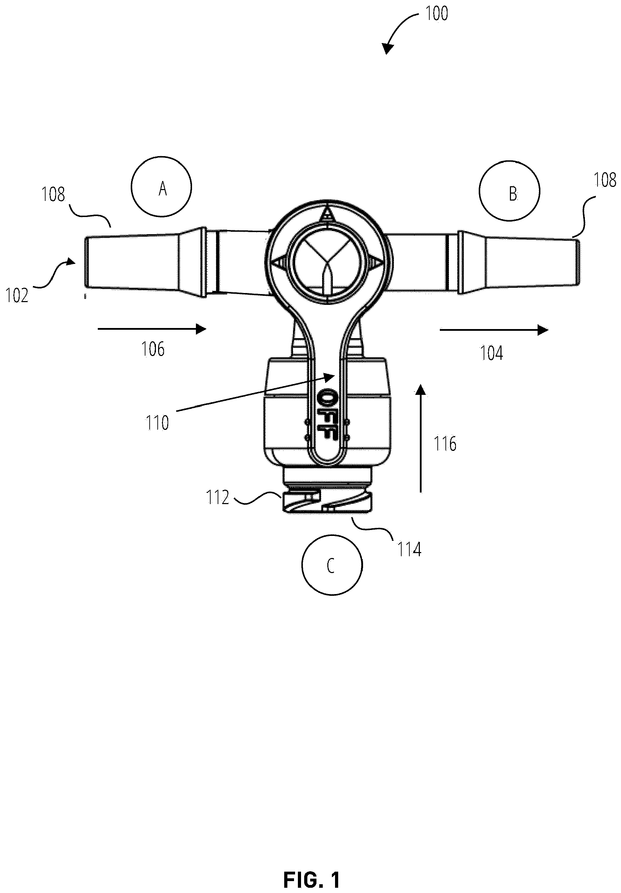

According to an aspect of the invention, the multi-port stopcock device preferably has the following elements and/or features, as shown in FIG. 1 - FIG. 6: at least one high-flow, swabable 114, female self sealing luer tip 112 stopcock for the control of biofluids, several ports for the flow of biofluids and/or calibration fluids, including but not limited to an inlet port (A), an outlet port (B), and a calibration port (C), a selectively pivotable lever 110 for the control of the flow of biofluids and/or calibration fluids.

According to an aspect of the invention, the use of high-flow, swabable, luer-lock stopcocks with swabable 114 and/or female self sealing luer tips 112 preferably controls flow of biofluids from patient to sensor, sensor to calibration port. The high flow stopcock preferably minimizes chances of blockages while allowing fluid flow control in an intuitive manner.

Further, the stopcocks in one embodiment may be designed to have 2 possible configurations to prevent possibility of reference materials from entering the patient.

Alternatively, in another embodiment, an adapter may preferably be placed on top of an off-the-shelf stopcock to prevent the possibility of reference materials from entering the patient. An adapter may limit movement of a stopcock lever in order to control the movement of reference materials, and/or patient biofluids.

In accordance with different embodiments, any number of luer-lock syringes may be employed in order to deliver reference materials and/or calibration fluids to the sensor. This preferably allows for any number of sensors to be calibrated using any number of reference materials.

Further, the use of non-toxic reference materials that have a rinsing property and will not affect the performance of the sensor may be employed. This preferably allows for in-situ cleaning of the sensors that have been exposed to patient biofluids before calibration, ensuring the best accuracy possible.

According to an aspect of one preferred embodiment, the selectively pivotable lever 110 preferably allows or restricts the flow of calibration fluids, cleaning fluids, and/or reference materials, from port (C) to one or more sensors.

Further sensors may be provided within the stopcock body or ports for detecting relevant environmental conditions including but not limited to flow, bubbles, types of fluid, and/or whether or not syringes/tubing are connected.

The sensors may preferably employ capabilities to transmit sensor readings to external device such as tablets or smartphones.

Sensors may be in the stopcocks 604, 606, and/or they can be downstream of the calibration port (C) in the Flow cell 510.

Flow cells may further comprise additional features such as internal vibration motors for breaking up bubbles in the flow cell.

According to an aspect of one preferred embodiment, turning of the lever 110 may comprise a manual mechanism or automatic mechanism.

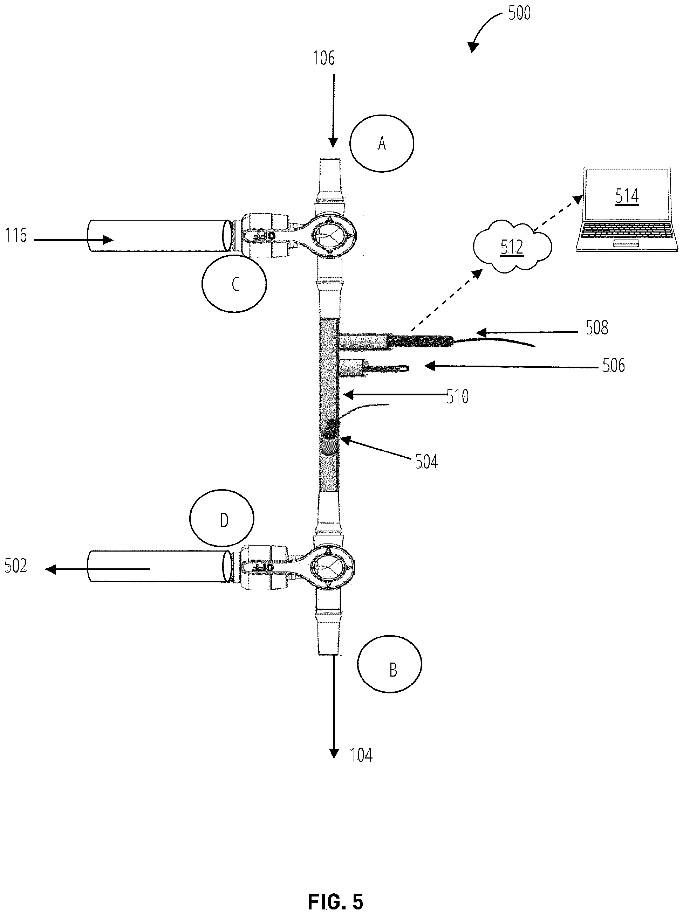

According to an aspect of the invention, and visualized in FIG. 5 - FIG. 6, there is preferably provided a flow cell, which is attached to the stopcock via catheter/tubing.

As may be best appreciated in view of FIG. 5 - FIG. 7, there is disclosed the use of the multi-port stopcock in combination with the flow cell, wherein the stopcock is preferably attached upstream, between the flow cell and the patient.

According to an aspect of one preferred embodiment, and visualized in FIG. 1 - FIG. 4, there may be provided a three-way stopcock configuration, comprising: an inlet port (A), an outlet port (B), and a calibration port C).

According to an aspect of another preferred embodiment, and visualized in FIG. 5 -FIG. 7, there may be provided a four-way stopcock configuration, comprising: an inlet port (A), an outlet port (B), a calibration port (C), and a calibration fluid outlet port (D), for selectively diverting the flow of calibration fluids from outlet port (B) to waste.

According to an aspect of the disclosure, the use of the multi-port stopcock in combination with the flow cell preferably enables: a) in-situ monitoring of patient fluids and/or exudates: at the inlet port (A) patient fluid flow in 106 leads to the flow cell, where the Biosensor- e.g. Electrical conductivity (EC) 504, biosensor e.g. pH reference electrodes 506, Biosensor e.g. pH electrode 508 may continuously monitor patient fluids as the patient fluid flow out 104 exits at the outlet port (B). Preferably, the default mode for the multi-port stopcock would be to allow fluid flow between the patient and the flow cell. b) In-situ calibration and/or cleaning of sensors: at the calibration port C, calibration/cleaning fluid flow in 116 leads to the flow cell, where where the Biosensor- e.g. Electrical conductivity (EC) 504, biosensor e.g. pH reference electrodes 506, Biosensor e.g. pH electrode 508 may continuously monitor patient fluids as the calibration/cleaning fluid flow out 502 exits at the calibration fluid outlet port (D), calibration/cleaning fluid flow out 502 which preferably lead to a waste reservoir.

The lever 110 may be oriented during the in-situ calibration/cleaning, to block off the inlet port (A) and allow only flow (calibration/cleaning fluid flow in 116) from the calibration port (C), and re-oriented afterwards for flow of allowing patient fluids to resume flow out through the outlet port B.

Alternatively, the lever 110 may be oriented during the in-situ calibration/cleaning to preferably allow the patient fluid flow in 106 and the calibration/cleaning fluid flow in 116 to flow from port (A) and (C), respectively, to the calibration/cleaning outlet port (D).

In some embodiments, there may be provided automated control of stopcock or other flow control mechanism (valves, etc.), for the automatic control of aspects including, but not limited to, automated fluid delivery, number of reference fluids delivered per sensor, number of sensors calibrated , amount of fluid delivered, volume of syringe used, type of syringe used, order of fluids delivered , ratio of reference fluid:air or reference fluid:rinsing agents, type of rinsing agents incorporated into the reference fluids, combinations of reference fluids to calibrate multiple sensors, incorporation of stopcock or other flow control mechanisms into the flow cell, use of other flow control mechanisms other than stopcocks, incorporation of algorithms that can detect the different reference fluids and automatically control the calibration process.

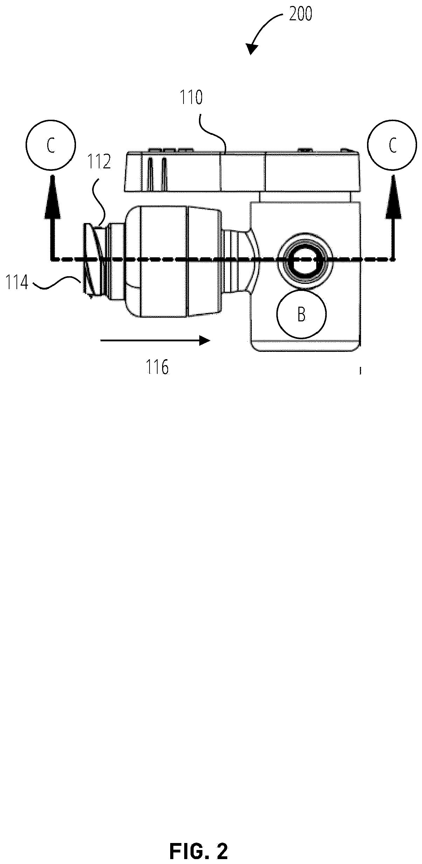

As may be best appreciated in view of FIG. 2, where the cross-section of port 1 is shown, the inner diameters are preferably consistent with one another.

Devices and methods for carrying out the invention are presented in terms of embodiments depicted within the FIGS. However, the invention is not limited to the described embodiments, and a person skilled in the art will appreciate that many other embodiments of the invention are possible without deviating from the basic concept of the invention, and that any such work around will also fall under scope of this invention. It is envisioned that other styles and configurations of the present invention can be easily incorporated into the teachings of the present invention, and the configurations shall be shown and described for purposes of clarity and disclosure and not by way of limitation of scope.

The features of the invention and disclosure which are believed to be novel are particularly pointed out in the specification. The present invention now will be described more fully hereinafter with reference to the accompanying drawings, which are intended to be read in conjunction with both this summary, the detailed description and any preferred and/or particular embodiments specifically discussed or otherwise disclosed. The systems, devices and methods of the present disclosure may, however, be embodied in many different forms and should not be construed as limited to the embodiments set forth herein; rather, these embodiments are provided by way of illustration only and so that this disclosure will be thorough, complete and will fully convey the full scope of the invention to those skilled in the art.

FIG. 1 illustrates a top view of a multi-port stopcock device-three-port embodiment 100.

In the illustrated embodiment, a three-way stopcock device is shown, comprising three ports: an inlet port (A), an outlet port (B), and a calibration port (C), according to an embodiment of the invention.

An inlet portion (A) further comprising patient fluid entry 102, which flows in from the male barbed slip tip 108 in the direction of patient fluid flow in 106.

The default mode for the patient fluid flow out occurs at the outlet port (B), further comprising patient fluid flow out 104, where the outlet port (B) typically flows back to the patient through the male barbed slip tip 108.

A second mode for fluid flow occurs at the calibration port (C), further comprising the female self sealing luer tip 112/ swabable tip 114, where a calibration/cleaning fluid flow in 116 flows from port (C) to an outlet port.

A mechanism comprising a portion, lever 110, controls the flow of the patient fluid and calibration/cleaning fluid. As shown in FIG. 1, the lever portion is "off' to the calibration/cleaning fluid, allowing only the patient fluid flow in 106 to flow through to the patient fluid flow out 104.

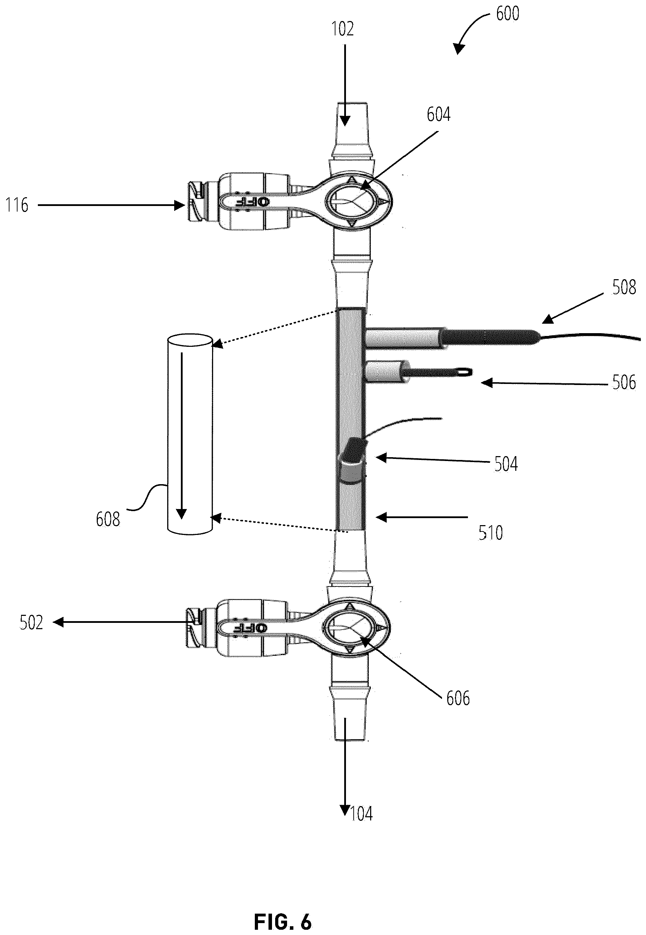

Opening the lever 110 to port (C) allows the calibration/cleaning fluid flow in 116 to flow from port (C) through the female self sealing luer tip 112/ swabable tip 114, through to the patient fluid flow out 104 (in a three-port embodiment), or to an additional waste port (which can be seen in FIG. 6- FIG. 7.

The lever 110 may be pivoted between positions manually or remotely via an electronic mechanism.

FIG. 2 illustrates a side view of the multi-port stopcock device- three-port embodiments 200 device, viewed along the axis of the outlet port (B).

In this embodiment, the lever 110 is open to port (C), further comprising a female self sealing luer tip 112/ swabable tip 114, allowing the calibration/cleaning fluid flow in 116 from port (C).

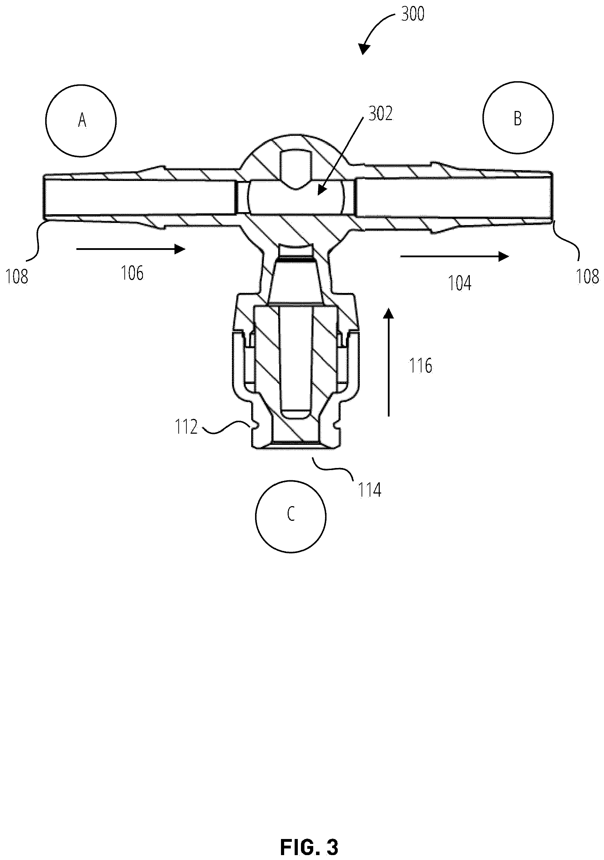

FIG. 3 illustrates a cross-sectional view of the multi-port stopcock device- three-port embodiment 300 illustrating the mechanism for flow control of the lever 110, namely a rotatable t-channel 302, which blocks or allows fluid to flow.

In the embodiment shown in FIG. 3, the rotatable t-channel 302 is open to port (A), allowing patient fluid entry 102 to flow from patient fluid flow in 106 to Port (B), and out through patient fluid flow out 104.

In the embodiment shown in FIG. 3, the rotatable t-channel 302 is closed to port (C), blocking calibration/cleaning fluid flow in 116 from entering.

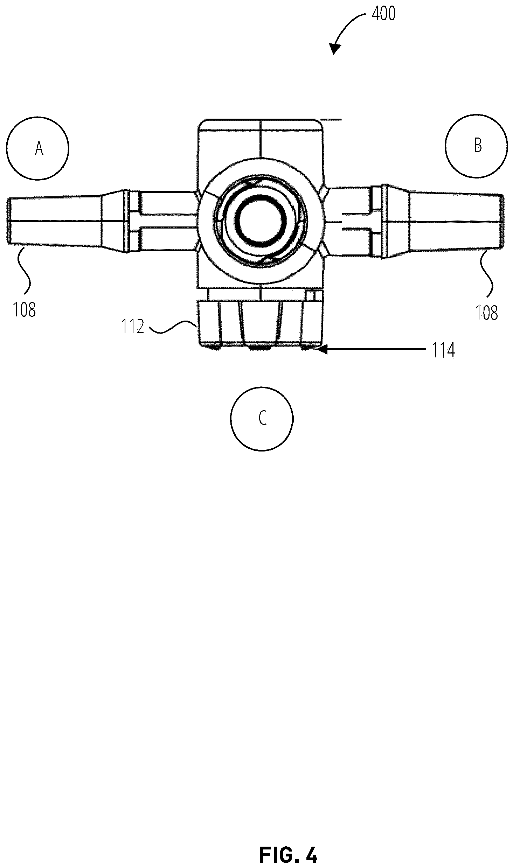

FIG. 4 illustrates a rear view of a three-port stopcock device, viewed along the axis of the calibration port (C). The inlet and outlet ports of Ports A and B, respectively connect to fluid sources or reservoirs via male barbed slip tips 108. Calibration or cleaning fluid may flow in through a syringe, connectable to Port C of the stopcock device via the female self sealing luer tip 112. Port C may alternatively comprise a swabable tip 114, or a combination of luer and swabable tips 112, 114.

FIG. 5 illustrates a multi-port stopcock flow cell device 500, embodiment comprising an inlet port (A), an outlet port (B), a calibration/cleaning port (C), a calibration/cleaning outlet port (D), connected by means of a Flow cell 510, which comprises multiple biosensors 504, 506, 508, the biosensors capable of measuring fluid properties.

Generally, the multiport stopcock device preferably comprises: one or more inlet ports(A,C), receiving fluid from a fluid source, fluidically connected by a flow cell 510 comprising one or more fluid channels(shown in FIG. 6-7), to one or more outlet ports(B,D); and a mechanism (i.e. the lever 110) for diverting flow of fluid between ports and fluid channels, wherein: at least one of the one or more inlet ports comprises an inlet tip, connectable to a syringe, and a slip tip, connectable to said fluid source, and at least one of the one or more outlet ports comprises an inlet tip, connectable to a syringe, and a slip tip, connectable to a reservoir.

In this embodiment, flow of the patient fluid flow in 106 and calibration/cleaning fluid flow in 116 enter from ports (A) and (C), respectively, by means of a lever 110, located at the junction between ports (A) and (C) as in the previously described embodiment.

Further in this embodiment, the patient fluid flow out 104and calibration/cleaning fluid flow out 502 are controlled by an additional lever 110 located at the junction between ports (B) and (D).

Upon fluid flowing through the flow cell 510, and over the biosensors 504, 506, 508, the biosensors measure data relating to fluid properties, and send the measured data, via a connection mechanism 512, to a computing device 524.

The connection mechanism 512 may be wired or wireless, and may include, but is not limited to, the internet, a wired connection, Bluetooth, NFC, and any other connection mechanisms known in the art. The data may be sent to cloud storage, and downloadable to the computing device 524. The computing device 524 may comprise a computer, laptop, smart phone, tablet, or any other computing device known in the art. The computing device 524 may be on-site, with the multi-port stopcock flow cell device 500, or remote from it. In an example, data may be measured at a patient's home, and automatically sent, via the internet, to a hospital or lab environment for analysis.

FIG. 6 illustrates a multi-port stopcock flow cell device- four port, one channel embodiment 600 comprising an inlet port (A), an outlet port (B), a calibration/cleaning port (C), a calibration/cleaning outlet port (D), with stopcocks 604 and 606 located at the inlet ports and the outlet ports, connected by means of a Flow cell 510, which comprises multiple biosensors 504, 506, 508, wherein the flow of either the patient fluid flow in 106 or the calibration/cleaning fluid flow in 116 is directed along Path 1 - over Path 1- over the Path 1 - over biosensors 602.

Internal view- fluid channel 608 illustrates one fluid channel, comprising a path 1 - over biosensors 602, for fluids to flow through.

Fluids pass through the entry stopcock 604 from patient fluid entry 102 or from calibration/cleaning fluid flow in 116, according to whether stopcock device is in calibration mode or non-calibration mode.

### Non-calibration mode:

1. entry stopcock 604 set to block calibration/cleaning fluid flow in 116.
2. patient fluid flows in 106, Path 1 - over Path 1- over the Path 1 - over biosensors 602
3. exit stopcock 606 set to block calibration/cleaning fluid flow out 502.
4. patient fluid flow out 104.

### Calibration mode:

1. Entry stopcock 604 set to block patient fluid entry 102.
2. Calibration/cleaning fluid flow in 116, Path 1 - over biosensors 602.
3. Exit stopcock 606 set to flow calibration fluid through to calibration/cleaning fluid flow out 502.

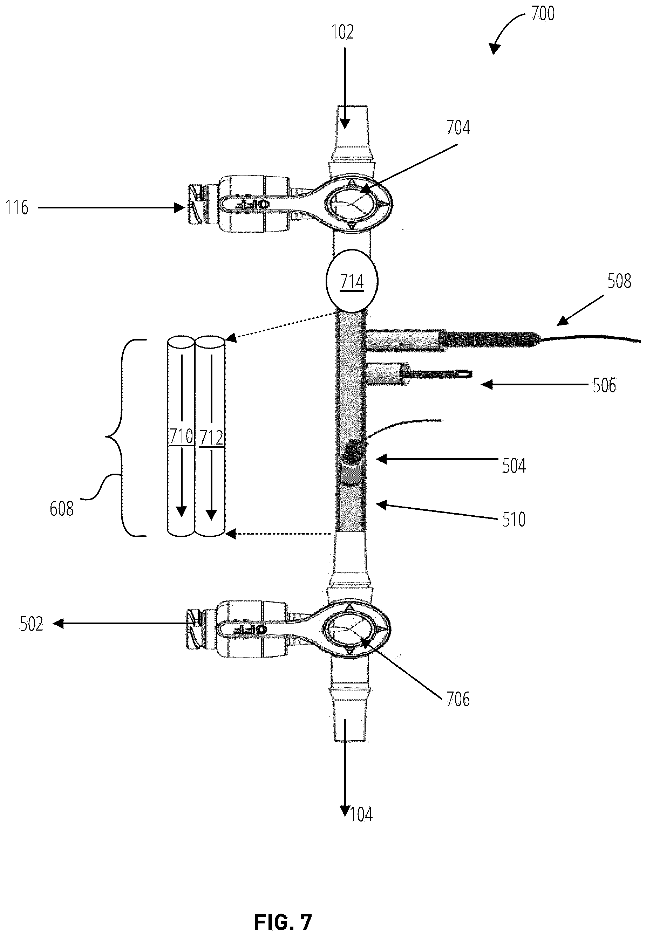

FIG. 7 illustrates a multi-port stopcock flow cell device- four port, two channel embodiment 700, comprising an inlet port (A), an outlet port (B), a calibration/cleaning port (C), a calibration/cleaning outlet port (D), with stopcocks 604 and 606 located at the inlet ports and the outlet ports, connected by means of a Flow cell 510, which comprises multiple biosensors 504, 506, 508, wherein the flow of either the patient fluid flow in 106 or the calibration/cleaning fluid flow in 116 is directed along either or both of two channels- Path 1-over the biosensors 710, and Path 2- bypass 712.

Fluids pass through the entry stopcock 604 from patient fluid entry 102 or from calibration/cleaning fluid flow in 116, and are either passed through the Flow cell 510, over the biosensors, to the patient fluid flow out 104 or through the Flow cell 510, to Path 1 - over biosensors 602,to the calibration/cleaning fluid flow out 502.

### Non-calibration mode:

1. entry stopcock 604 set to block calibration/cleaning fluid flow in 116.
2. patient fluid flows in 106, Path 1 - over biosensors 602
3. Exit stopcock 606 set to block calibration/cleaning fluid flow out 502, calibration/cleaning fluid flow in 116 directed along Path 2- bypass 712 to calibration/cleaning fluid flow out 502.
4. patient fluid flow out 104.

### Calibration mode:

1. Entry stopcock 704 set to block patient fluid entry 102.
2. Calibration/cleaning fluid flow in 116, Path 1 - over biosensors 602.
3. Exit stopcock 606 set to flow calibration fluid through to calibration/cleaning fluid flow out 502.
4. Patient fluid flow in 106 directed along Path 2- bypasses 712 to patient fluid flow out 104.

In some embodiments, the flow cell may be equipped with a bubble removal means 714.

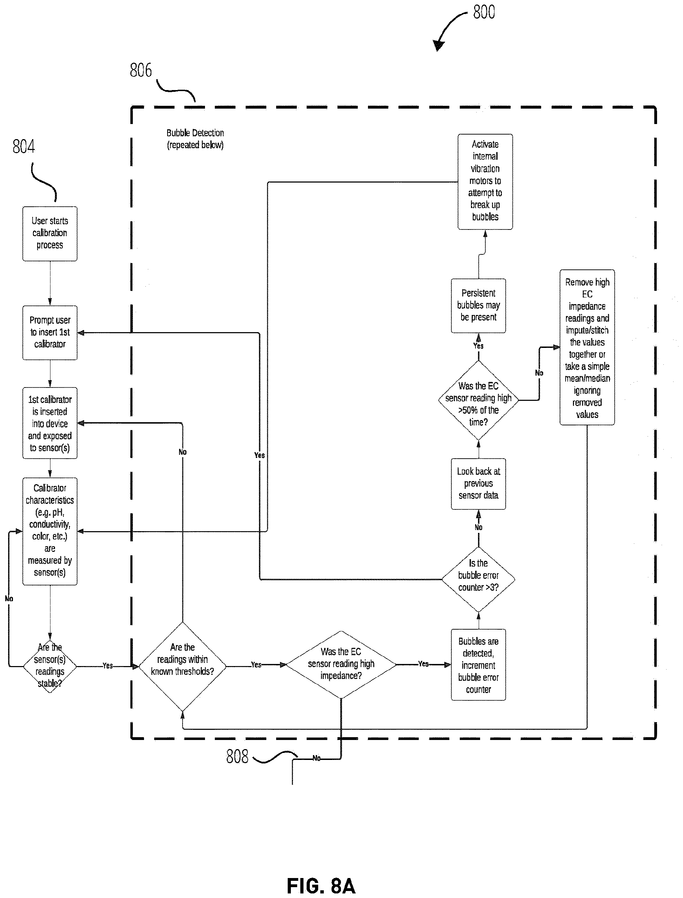

The computing device 524 may be communicatively coupled with an active bubble removal means 714, including, but not limited to, a vibration motor, ultrasonicating device, or the like, where the active bubble removal means may be within the stopcock or the flow cell (or both), and may be automatically activated by the method, such that the vibration of the motor breaks up the bubbles. Other active techniques, communicatively to the computing device, may include optical, electric, mechanical, or thermal fields, or combinations thereof. This embodiment is discussed in greater detail with respect to FIG. 8A.

Alternatively, or in combination, there may be provided a passive bubble removal means 714, including, but not limited to, bubble traps on or in the flow cell, or at inlet ports of the flow cell. Bubble traps may be used in combination with vacuum pumps if a sample needs to be degassed. Bubble traps may be commercially available or may be formed by adjusting the geometry of the inlet ports or flow cells.

In either calibration or non-calibration mode, the signals measured by the sensors are sent to a computing device (via, for example, the internet, a wired connection, bluetooth, NFC, and any other connection mechanisms known in the art).

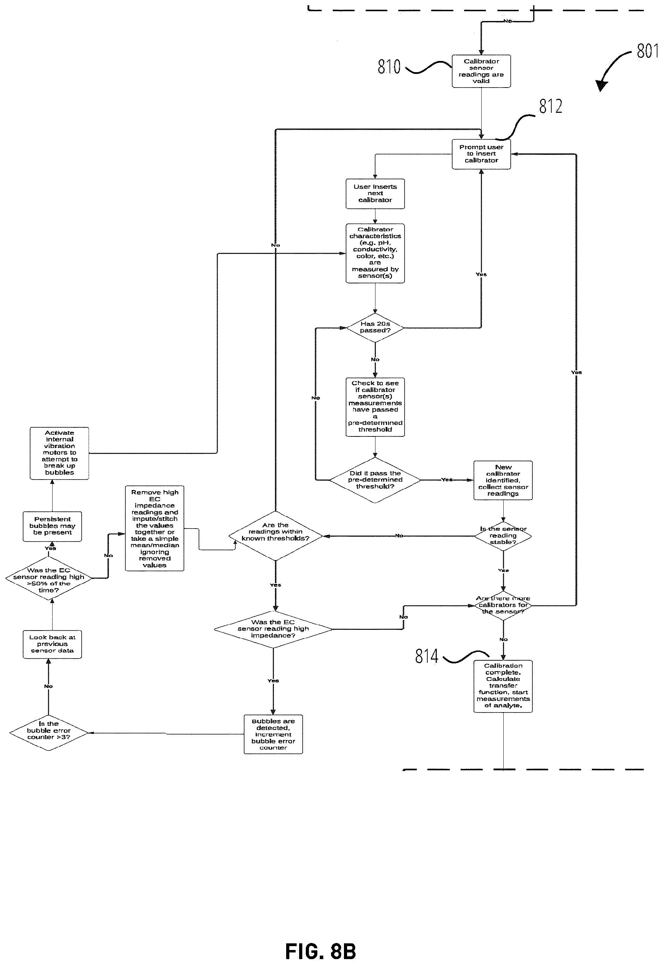

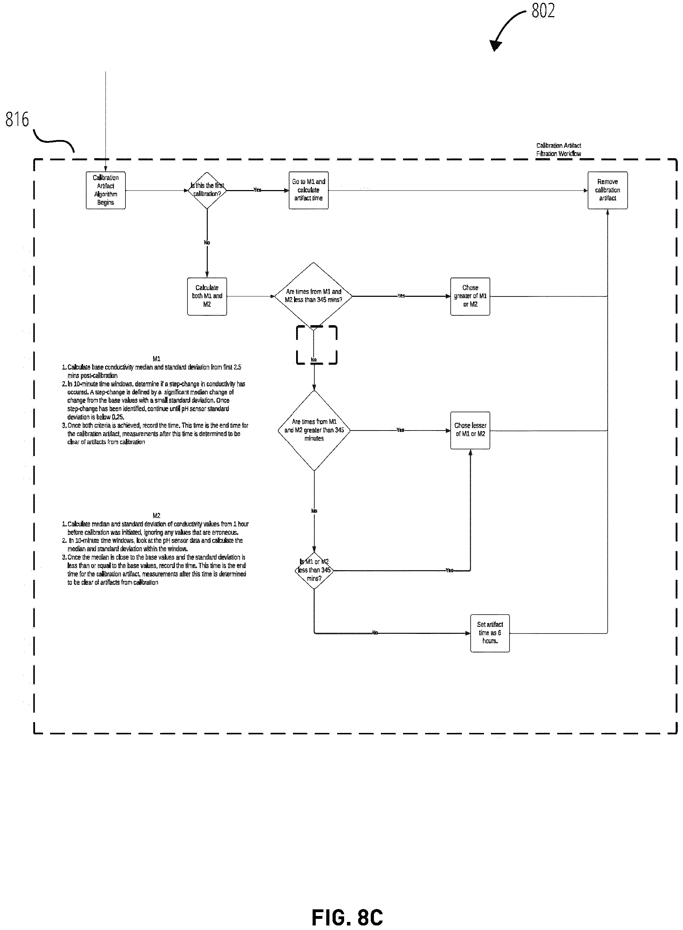

FIGS 8. A-C comprise flow charts relating to Data validation 800, and applications of the methods.

FIGS, 8 A-C relate to EC and pH sensor calibration methods. It should be readily understood that this method may be applied to calibration of other sensors that require external calibrators, such as UV-VIS, optical sensors, lactate, glucose, and the like.

Instructions for performing the method may be stored on a computer readable, non-transitory medium, that, when executed by a computer, cause it to perform the steps described below.

Broadly, the steps may be described as two alternating steps, and a third step. Alternating data validation (FIG. 8A) and data calibration (FIG. 8B) steps are performed each pair of validation and calibration steps corresponding to a calibration fluid flowing through the flow cell and being measured by the sensors 504- 508, in the flow cell 510. The calibration data is then filtered/ augmented in a third, filtering/augmentation step (FIG. 8C). For multiple types of calibration fluids, the third step may be performed after all of the data validation and calibration steps.

Generally, the flow charts in FIG. A-C relate to methods of in-situ calibration.

The method for in-situ calibration of a multi-port stopcock flow cell generally comprises executing, by a computing device, instructions stored on the memory, which cause the processor to perform the steps:
1. prompt a user to insert a calibration fluid into an input port of a multiport stopcock device, the calibration fluid flow diverted via a mechanism for diverting flow, through a flow cell comprising one or more fluid channels, the flow cell fluidically connected to the multiport stopcock device, the multiport stopcock device comprising one or more sensors for measuring data relating to the calibration fluid;
2. receive, via a connection mechanism connecting the one or more sensors to the computing device, the measured data;
3. determine whether the measured data is valid, and upon the data being valid, repeat steps 1-3 until the user indicates that there are no more calibration fluids;
4. Filter the calibration data based on pre-determined thresholds.

In view of the specific example of conductivity in FIGS. 8A-C, the method involves the following:

### FIG. 8A: Data validation 800

1. execution 804: the method first prompts a user to insert a first calibrant. Referring to FIGS. 5-7, the user may flow calibration fluid through port C, and divert the stopcock such that the calibration fluid flows over the biosensors, comprises one or more instances of a user inserting a calibrator into the calibration port of the stopcock device, the sensors in the flow cell measure data relating to the calibrant (for example, conductivity of a buffer solution) and send the data to the computing device.
2. bubble detection 806: ensuring stable readings, determining whether bubbles are detected in the flow cell by abnormal readings, such as high Electrical Conductivity (EC) readings. Abnormal readings may be pre-set thresholds, or may be learned by the method based on the average (or other metric) of other readings. Upon determining that readings are abnormal and the data is invalid, the method may perform corrective actions, such as activating an active bubble removal means (see FIG. 7).
3. next step 808: the readings are stable, within thresholds, and are not abnormal, the calibration data may be sent to the next step (described in FIG. 8B).

### FIG. 8B: data calibration 801

4. Following the data validation step, valid data saved for processing 810.
5. calibration prompt 812 prompts a user to insert another calibrator. If there is another calibrator, repeat steps 1-3 of FIG. 8A. Otherwise, proceed to next step.
6. calibration complete 814 when there are no more calibrants to be added.

### FIG. 8C: calibration artifact filtration 802

5. Following the calibration and data validation steps, the valid data that was saved for processing from step 4, is filtered via the calibration artifact filtration 802, based on pre-set thresholds, including, but not limited to, one or more of: time, stability, standard deviation, mean, median, and the like.
6. In this embodiment, the example relates to EC and pH, with thresholds M1 and M2.
   1. M1:
      1. Calculate base conductivity median and standard deviation from a first time threshold past calibration (i.e. 2.5 minutes), or after a number of samples (i.e. after 5 calibration samples have been run).
      2. In a pre-set time window (i.e. 10 minutes), determine if step-change in conductivity (significant median of change from base values, with small standard deviation) occurred. Then continue until pH sensor standard deviation is below a threshold (in this case 0.25).
      3. Once both criteria are achieved, record time- this is the end time for the calibration artifact: measurements after the end time are determined to be clear of artifacts from calibration.
   2. M2:
      1. Calculate median and standard deviation of conductivity values from a time before initiating calibration (i.e. 1 hour), ignoring erroneous values.
      2. In pre-set time windows (i.e. 10 minutes), calculate median and standard deviation of data within the window.
      3. When the median is close to the base values and the standard deviation is less than or equal to the base values, record the time. This time is the end time for the calibration artifact- measurements after this time are determined to be clear of artifacts from calibration.

The method may further employ various decision making engines which evaluate the calibration data in order to decide which data may be best suited for determining calibration artifacts. For example, very high or very low pH or conductivity may be filtered out of the calibration, and data determined to be invalid due to the presence of bubbles may be filtered out.

Calibrated data from the method disclosed above is preferably used to process fluid data, i.e. from a patient.

The multiport stopcock device may be in fluid communication with a patient, such as by connection to a patient catheter, for monitoring patient biofluids. Using the multi-port stopcock flow cell device allows for multiple sensor calibrations and readings, without disconnecting the device from the patient. The device may alternatively be calibrated elsewhere, and measurements may be performed at the point of care, i.e. at the patient's home. Additional sensor readings may relate to pH, lactate, amylase, urea, creatinine, electrical conductivity, light absorbance, and/or colour.

Machine learning algorithms may be applied to previously acquired signal data associated with a user condition or calibration anomalies. For example, pattern recognition may be performed on previously acquired signal data that is associated with a particular user condition. The machine leaning may generate a user condition classification model trained by the previously acquired signal data.

These may include, for example, deep learning architectures such as Deep Belief Network (DBN), Stacked Auto Encoder (SAE), Convolutional Neural Network (CNN) or Recurrent Neural Network (RNN) may be used. Other examples include, without limitation, Restricted Boltzmann machines (RBM), Social Restricted Boltzmann Machines (SRBM), Fuzzy Restricted Boltzmann Machines (FRBM), TTRBM models of Deep Belief Networks (DBN) or similar approaches could be used; AE, FAE, GAE, DAE, BAE models of Statistically Adjusted End Use (SAE) models could be used; models such as AlexNet, ResNet, Inception, VGG16, ECNN models of CNN may be used; Bidirectional Recurrent Neural Networks (BiRNN), Long Short-Term Memory (LSTM) networks, Gate Recurrent Unit (GRU) of RNN may also be used. Additional techniques specific to time-series modelling may be employed, including, but not limited to, dynamic time warping, change point detection, Autoregressive Integrated Moving Average (ARIMA).

In some embodiments, other types of algorithms such as physics-based mathematical computations and basic multiple linear regression models may also be relied upon in conjunction with or in complementarity with those architectures and learning algorithms. This may further include cumulative average (CA) methods.

The present disclosure includes systems having processors to provide various functionality to process information, and to determine results based on inputs. Generally, the processing may be achieved with a combination of hardware and software elements. The hardware aspects may include combinations of operatively coupled hardware components including microprocessors, logical circuitry, communication/networking ports, digital filters, memory, or logical circuitry. The processors may be adapted to perform operations specified by a computer-executable code, which may be stored on a non-transitory computer readable medium.

The steps of the methods described herein may be achieved via an appropriate programmable processing device or an on-board field programmable gate array (FPGA) or digital signal processor (DSP), that executes software, or stored instructions. In general, physical processors and/or machines employed by embodiments of the present disclosure for any processing or evaluation may include one or more networked or non-networked general purpose computer systems, microprocessors, field programmable gate arrays (FPGA's), digital signal processors (DSP's), micro-controllers, and the like, programmed according to the teachings of the exemplary embodiments discussed above and appreciated by those skilled in the computer and software arts. Appropriate software can be readily prepared by programmers of ordinary skill based on the teachings of the exemplary embodiments, as is appreciated by those skilled in the software arts. In addition, the devices and subsystems of the exemplary embodiments can be implemented by the preparation of application-specific integrated circuits, as is appreciated by those skilled in the electrical arts. Thus, the exemplary embodiments are not limited to any specific combination of hardware circuitry and/or software.

Stored on any one or a combination of computer readable media or non-transitory computer readable media, the exemplary embodiments of the present invention may include software for controlling the devices and subsystems of the exemplary embodiments, for processing data and signals, for enabling the devices and subsystems of the exemplary embodiments to interact with a human user or the like. Such software can include, but is not limited to, device drivers, firmware, operating systems, development tools, applications software, and the like. Such computer-readable media further can include the computer program product of an embodiment of the present invention for preforming all or a portion (if processing is distributed) of the processing performed in implementations. Computer code devices of the exemplary embodiments of the present invention can include any suitable interpretable or executable code mechanism, including but not limited to scripts, interpretable programs, dynamic link libraries (DLLs), complete executable programs and the like.

Common forms of computer-readable media may include, for example, magnetic disks, flash memory, RAM, a PROM, an EPROM, a FLASH-EPROM, or any other suitable memory chip or medium from which a computer or processor can read.

While the present disclosure describes various embodiments for illustrative purposes, such description is not intended to be limited to such embodiments. On the contrary, the applicant's teachings described and illustrated herein encompass various alternatives, modifications, and equivalents, without departing from the embodiments, the general scope of which is defined in the appended claims. Information as herein shown and described in detail is fully capable of attaining the above-described object of the present disclosure, the presently preferred embodiment of the present disclosure, and is, thus, representative of the subject matter which is broadly contemplated by the present disclosure.

## Claims

1. A multiport stopcock device comprising:
one or more inlet ports, receiving fluid from a fluid source, the one or more inlet ports fluidically connected by a flow cell comprising one or more fluid channels, to one or more outlet ports; and
a mechanism for diverting flow of fluid between ports and fluid channels,
wherein: at least one of the one or more inlet ports comprises an inlet tip, connectable to a syringe, and a slip tip, connectable to said fluid source, and at least one of the one or more outlet ports comprises an inlet tip, connectable to a syringe, and a slip tip, connectable to a reservoir.

2. The multiport stopcock device of claim 1, wherein:
at least one of the one or more inlet ports comprises an inlet port having a swabable or self-sealing luer tip, connectable to a calibration fluid source, and a male barbed slip tip, connectable to a female end of a patient fluid source.

3. The multiport stopcock device of claim 1, wherein said fluid source comprises one or more of a calibration fluid, a cleaning fluid, and a biofluid from a patient.

4. The multiport stopcock device of claim 1, further comprising a bubble removal means which actively or passively removes bubbles from the flow cell.

5. The multiport stopcock device of claim 3, wherein the reservoir comprises a fluidic connection to the patient.

6. The multiport stopcock device of claim 3, wherein the reservoir comprises a waste reservoir.

7. The multiport stopcock device of claim 1, the flow cell further comprising:
one or more sensors in fluid communication with the one or more fluid channels, the sensors communicatively coupled to a computing device.

8. The multiport stopcock device of claim 7, the sensors comprising one or more of: pH, lactate, amylase, urea, creatinine, electrical conductivity, light absorbance, and/or colour sensors.

9. The multiport stopcock device of claim 1, wherein the mechanism for diverting flow may be equipped with automatic and/or remotely controlled capabilities, and may comprise a mechanical or electrical mechanism.

10. A method for in-situ calibration of a multi-port stopcock flow cell, the method comprising:
executing, by a computing device, instructions stored on the memory, which cause the processor to perform the steps:
1. prompt a user to insert a calibration fluid into an input port of a multiport stopcock device, the calibration fluid flow diverted via a mechanism for diverting flow, through a flow cell comprising one or more fluid channels, the flow cell fluidically connected to the multiport stopcock device, the multiport stopcock device comprising one or more sensors for measuring data relating to the calibration fluid;
2. receive, via a connection mechanism connecting the one or more sensors to the computing device, the measured data;
3. determine whether the measured data is valid, and upon the data being valid, repeat steps 1-3 until the user indicates that there are no more calibration fluids;
4. Filter the calibration data based on pre-determined thresholds.

11. The method of claim 10, the sensors comprising one or more of: pH, lactate, amylase, urea, creatinine, electrical conductivity, light absorbance, and/or colour sensors.

12. The method of claim 10, wherein the mechanism for diverting flow may be equipped with automatic and/or remotely controlled capabilities, and may comprise a mechanical or electrical mechanism.

13. The method of claim 10, the method further comprising the step 3.b:
upon the data not being invalid, perform corrective action in order to obtain valid data.

14. The method of claim 13, wherein invalid data comprises high electrical conductivity readings caused by one or more bubbles in the flow cell.

15. The method of claim 14, wherein corrective action comprises activating an active bubble removal means coupled to the flow cell, that, when activated, actively removes bubbles from the flow cell.

16. The method of claim 10, the pre-determined thresholds comprising data in a pre-set time window being below pre-set standard deviations and above pre-set step changes.

17. A computer readable, non-transitory storage medium, comprising instructions that, when executed by a processor, causes the processor to perform the steps of:
1. prompt a user to insert a calibration fluid into an input port of a multiport stopcock device, the calibration fluid flow diverted via a mechanism for diverting flow, through a flow cell fluidically connected to the multiport stopcock device, comprising one or more sensors for measuring data relating to the calibration fluid;
2. receive, via a connection mechanism connecting the one or more sensors to the computing device, the measured data;
3. determine whether the measured data is valid, and upon the data being valid, repeat steps 1-3 until the user indicates that there are no more calibration fluids;
4. Filter the calibration data based on pre-determined thresholds.

18. The computer readable, non-transitory storage medium of claim 17 the sensors comprising one or more of: pH, lactate, amylase, urea, creatinine, electrical conductivity, light absorbance, and/or colour sensors.

19. The computer readable, non-transitory storage medium of claim 17, wherein the mechanism for diverting flow may be equipped with automatic and/or remotely controlled capabilities, and may comprise a mechanical or electrical mechanism.

20. The computer readable, non-transitory storage medium of claim 17, the pre-determined thresholds comprising data in a pre-set time window being below pre-set standard deviations and above pre-set step changes.
